# EUROPÄISCHE PATENTSCHRIFT

(11) **EP 1 358 926 B1**
(45) Veröffentlichungstag und Bekanntmachung des Hinweises auf die Patenterteilung: **27.06.2007**
(21) Anmeldenummer: 02009658.2
(22) Anmeldetag: 29.04.2002
(51) Int. Cl.: B01D 69/12, B01D 71/06, C08J 5/22, C08K 5/00, C08K 5/5419, C08K 5/549

(54) **Erzeugnisse für den Gasaustausch und die Abtrennung biologischer Materialien oder die Trennung von Stoffgemischen und Verwendungen von Nanocomposites**
Devices for gas exchange and the separation of biological materials or the separation of mixtures and the use of nanocomposites
Dispositifs d'échange gazeux et la séparation de matières biologique ou la séparation de mélanges de substances et l'utilisation de nanocomposites

(43) Veröffentlichungstag der Anmeldung: 05.11.2003
(73) Patentinhaber: EPPENDORF AG, 22339 Hamburg (DE)
(72) Erfinder: Taesler, Christian, Dr. rer. nat., 21614 Buxtehude (DE); Koehn, Heinz-Gerhard, Dr. rer-nat., 22397 Hamburg (DE)
(74) Vertreter: Hauck Patent- und Rechtsanwälte

(56) Entgegenhaltungen:
- WO-A-00/76634
- WO-A-01/72885
- WO-A-99/04891
- US-A- 5 484 867

## Beschreibung

Die Erfindung bezieht sich auf neue Formteile für den Gasaustausch und die Abtrennung von Mikroorganismen und/oder Biomaterialien.

Bei vielen Verfahren, die im Labor oder in der Produktion durchgeführt werden, ist es erforderlich, Mikroorganismen und/oder Biomoleküle ("biologisches Material") von oder aus der Umgebung abzutrennen, zugleich jedoch einen Gasaustausch mit der Umgebung zu ermöglichen. Es kann geboten sein, biologisches Material daran zu hindern, in die Umgebung einzutreten und/oder biologisches Material aus der Umgebung daran zu hindern, ein anderes biologisches Material oder sonstiges Material zu kontaminieren. Ferner kann es erforderlich sein, Gase, die von einem biologischen Material entwickelt werden, in die Umgebung abzuführen und/oder Gase aus der Umgebung dem biologischen Material zuzuführen. Anwendungsfälle im Labor sind die Bakterienanzucht, die Gefriertrocknung, die Trocknung biologischer Materialien, enzymatische Reaktionen, z.B. Oxidase, und chemische Reaktionen mit Gasaustausch.

Bereits bekannt ist, Gefäße für die Aufnahme von Proben biologischen Materials mit einem Membranverschluß zu versehen, der einen Gasaustausch mit der Umgebung ermöglicht. Der Gasaustausch ist jedoch durch die Beschränkung auf den Öffnungsquerschnitt des Gefäßes stark eingeschränkt. Die bekannten Membranmaterialien haben zudem den Nachteil, daß sie aufgrund einer makrokopischen Porosität im Bereich von 0,4 bis 1 µm eine Durchlässigkeit auch für biologisches Material (z.B. Biomoleküle, Viren und kleine Einzeller (Mycoplasten)) aufweisen. Außerdem können beim Befüllen und Entleeren, das bei geöffnetem Gefäß erfolgt, Kontaminationen eintreten.

Des weiteren ist es bekannt, daß Mikrotiterplatten mit Silikonmatten abgedeckt werden. Silikon weist eine erhöhte Gasdurchlässigkeit gegenüber anderen verbreiteten Gefäßmaterialien auf, wie beispielsweise PET. Der Gasaustausch erfolgt aber nur über die Abdeckung, der Rest der Mikrotiterplatte ist gasundurchlässig. Gefäße aus Silikon sind wegen ihrer Weichheit und Verzugsneigung für das Automatenhandling und Zentrifugieren ungeeignet.

Des weiteren gibt es für Zellkulturen Vorrichtungen, die ähnlich wie ein Diarähmchen aufgebaut sind. Diese haben zwischen einem Rahmen, der seitlich abdichtende Durchgänge für das Einspritzen bzw. Entnehmen einer Probe aufweist, zwei gasdurchlässige Membranen, die beispielsweise aus Polystyrol bestehen. Durch die Durchgänge wird eine Probe zwischen die Membranen eingegeben, so daß diese sich zwischen den Membranen ausbreitet. Diese Technik setzt verhältnismäßig große Flächen für den Gasaustausch voraus ist bezüglich des Handlings bzw. platzbedarfsbezogen auf das Probenvolumen nachteilig.

Ferner gibt es im Labor und in der Produktion vielfach ein Bedürfnis nach einfachen Verfahren zur Trennung von Stoffgemischen.

Die WO 00/76634 A1 bezieht sich auf eine Gastrenneinrichtung in Form eines permeablen porösen Materials zum Trennen einer Gasmischung durch wählbare Porengrößen, wobei Poren gebildet aus zumindest einer nanostrukturierten Komponente enthalten sind. Durch Auswahl der spezifischen nanostrukturierten Komponente kann das poröse Material so maßgeschneidert werden, daß es Poren einer vorbestimmten Größe enthält, welche bestimmte Gase durchlassen und den Durchgang anderer Gase verhindern. Das gasdurchlässige poröse Material kann so geformt werden, daß es den Konstruktionsvoraussetzungen der dem Material zugeordneten Apparatur entspricht. Bevorzugt wird das poröse Material zu einem Film geformt. Eine Apparatur zum Trennen einer Mischung von Gasen umfaßt eine Kammer aus einem gasundurchlässigen Material, wobei die Öffnung der Kammer mit einem porösen Film geformt aus dem durchlässigen porösen Material bedeckt ist.

Nachteilig bei der bekannten Gastrenneinrichtung ist zum einen die aufwendige Herstellung der Kammer, die zur Aufnahme der zu trennenden Gasgemische dient. Diese muß insbesondere aufgrund ihrer Mehrteiligkeit in mehreren Schritten hergestellt werden. Auch die Bestückung der Kammer mit dem porösen Film ist aufwendig. Außerdem ist die Gasdurchlässigkeit der bekannten Gastrenneinrichtung gering, weil sie nur im Bereich der Öffnung der Kammer erfolgt. Ferner ist die Gastrenneinrichtung auf die Trennung von Gasen beschränkt.

Davon ausgehend liegt der Erfindung die Aufgabe zugrunde, ein Behältnis mit einer Trenneinrichtung für andere Trennaufgaben zur Verfügung zu stellen, das einfacher herstellbar und handhabbar ist und eine höhere Gasdurchgängigkeit aufweist.

Die Aufgabe wird durch ein Formteil für den Gasaustausch und die Abtrennung von Mikroorganismen und/oder Biomolekülen gemäß Anspruch 1 gelöst.

Vorteilhafte Ausgestaltungen der Lösung sind in den Unteransprüchen angegeben.

Das erfindungsgemäße Formteil für den Gasaustausch und die Abtrennung von Mikroorganismen und/oder Biomolekülen hat mindestens eine Wand bestehend zumindest teilweise aus einem gasdurchlässigen und für Mikroorganismen und/oder Biomoleküle undurchlässigen Nanocomposite enthaltend mindestens ein Polymer und mindestens einen Käfigmoleküle umfassenden Nanofüllstoff, wobei das Formteil ein Behältnis mit mindestens einer eine Öffnung aufweisenden Aufnahme ist, die ganz oder teilweise von der Wand begrenzt ist, so daß es einen Gasaustausch ermöglicht, der nicht durch die Öffnung hindurchgeht.

Bei dem erfindungsgemäßen Formteil für den Einsatz in biologischen Verfahren besteht mindestens eine Wand zumindest teilweise aus einem gasdurchlässigen und für Mikroorganismen und/oder Biomoleküle undurchlässigen Nanocomposite enthaltend mindestens ein Polymer und mindestens einen Käfigmoleküle umfassenden Nanofüllstoff.

Nanocomposites sind Kunststoffe, die mit Nanofüllstoffen gefüllt sind. Ein Nanocomposite aus mindestens einem Polymeren und mindestens einem Käfigmoleküle umfassenden Nanofüllstoff hat zwar eine Gasdurchlässigkeit, ist jedoch für Mikroorganismen und/oder Biomoleküle undurchlässig. Bei den Mikroorganismen handelt es sich um Bakterien, Bakteriophagen, Viren, Pilze, Schimmel, menschliche, tierische oder Pflanzenzellen. Die Biomoleküle sind Proteine, Enzyme, DNA, RNA oder Fragmente derselben. Die Gase sind insbesondere Gase, die von Mikroorganismen und/oder Biomolekülen erzeugt oder verbraucht werden, insbesondere Methan, Sauerstoff, Kohlendioxid, Stickstoffe, Schwefelwasserstoff, können aber auch Gase aus beliebigen chemischen Reaktionen sein.

Bereits bekannt ist, daß ein Nanofüllstoff die Gasdurchlässigkeit eines Polymers verbessern kann. Überraschenderweise wurde mit der Erfindung festgestellt, daß ein Nanocomposite enthaltend mindestens ein Polymer und mindestens einen Käfigmoleküle umfassenden Nanofüllstoff nicht nur eine Durchlässigkeit für Gase aufweist, sondern auch aufgrund der molekularen Porengroße (beispielsweise ca. 0,8 nm) für Mikroorganismen und/oder Biomoleküle undurchlässig sind. Insofern unterscheiden sich die Nanocomposites von Materialien, die für herkömmliche Membranen zum Einsatz kommen. Letztere weisen Porengrößen von 400 Nanometer und darüber auf, die insbesondere für Viren (Durchmesser etwa 50 bis 150 Nanometer) und kleine Bakterien (z.B. Mycoplasmen mit Durchmessern von etwa 150 Nanometer) durchgängig sind. Dies gilt erst recht für Biomoleküle, die noch viel kleinere Abmessungen haben können. Bei den Nanocomposites betragen die maximalen Durchmesser der Käfigmoleküle derzeit etwa 2 Nanometer (bei Einsatz von POSS beispielsweise etwa 1 Nanometer), so daß sie den Durchgang selbst kleinster Mikroorganismen und/oder von Biomolekülen wirksam unterdrücken. Infolgedessen werden erfindungsgemäß Stoffgemische, mechanisch stabile Membranen und Formteile zur Verfügung gestellt, die ganz oder teilweise gasdurchlässig sind, biologische Materialien jedoch vor Kontaminationen schützen bzw. eine Kontamination durch biologische Materialien verhindern.

Bei dem Polymer kann es sich insbesondere um einen Thermoplasten, einen Elastomeren oder um einen Duromeren handeln. Der Nanofüllstoff ist bevorzugt ein polyhedrales Oligosilsesquioxan (POSS) mit organischen Seitengruppen, die die Kompatibilität zum Polymer bewirken. Insbesondere bei Thermoplasten kann sich die Kompatibilität in einer vollständigen physikalischen Durchmischbarkeit des Nanofüllstoffes mit den Thermoplasten im geschmolzenen Zustand oder in einer Lösung des Polymeren in einem Lösungsmittel zeigen. Insbesondere bei Duromeren kann sich die Kompatibilität des Nanofüllstoffes in der chemischen Verbindbarkeit mit anderen Gruppen des Duromers zeigen. Geeignete polyhedrale Oligosilsesquioxane und andere für die Verwirklichung der Erfindung geeignete Nanofüllstoffe sind in der WO 01/72885 A1 beschrieben, deren Inhalt durch Bezugnahme in die vorliegende Anmeldung einbezogen ist. Die Erfindung ist aber nicht beschränkt auf den Einsatz der dort beschriebenen Stoffzusammensetzungen, sondern bezieht Nanocomposites enthaltend Käfigmoleküle mit Gasdurchlässigkeit und Undurchlässigkeit für Mikroorganismen und/oder Biomoleküle im weitesten Sinne ein.

Da der für die Gasdurchlässigkeit und für die Undurchlässigkeit verschiedener biologischer Materialien maßgebliche Nanofüllstoff über die Auswahl der organischen Seitengruppen molekular so ausgelegt werden kann, daß eine Kompatibilität mit verschiedenen Polymeren erreicht wird, kann das Polymer für die Anwendung optimal gewählt werden. Dies gilt beispielsweise für Anwendungen im Bereich sogenannter Extremophiles (Einzelzeller, die unter extremen Bedingungen existieren), wo die Temperatur zwischen 0 und 110 °C und der pH zwischen 0 und 14 betragen kann. Man kann für diese Anwendungen gasdurchlässiges und für biologisches Material undurchlässiges Polypropylen (PP) als Basismaterial für das Nanocomposite verwenden.

Durch Compoundierung eines Thermoplasten oder eines nachträglich aushärtenden Elastomeren oder Duromeren mit einem oder mehreren kompatiblen polyhedralen Oligosilsesquioxanen mit organischen Seitengruppen kann - abhängig von der Größe der POSS-Käfigmoleküle und vom eincompoundierten Anteil der POSS-Derivate - ein thermoplastisch verarbeitbares Material mit hoher, einstellbarer Gasdurchlässigkeit hergestellt werden. Dies gilt jeweils für die Gase, die aufgrund ihrer Molekülgröße durch die verwendeten POSS-Derivate diffundieren können. Durch die freie Auswahl des Basismaterials, zu dem die POSS-Derivate durch Wahl geeigneter organischer Seitengruppen kompatibel gemacht werden, können gasdurchlässige und für Mikroorganismen und/oder Biomoleküle undurchlässige Materialien für verschiedenste Anwendungen hergestellt werden.

Bei dem erfindungsgemäßen Formteil für den Einsatz in Trennverfahren besteht mindestens eine Wand bzw. bevorzugt das gesamte Formteil zumindest teilweise aus einem eine unterschiedliche Durchlässigkeit für Gase und/oder Gase und Flüssigkeiten und/oder Gase und Feststoffe und/oder Flüssigkeiten und Feststoffe und/oder Flüssigkeiten aufweisenden Nanocomposite enthaltend mindestens ein Polymer und mindestens einen Käfigmolküle umfassenden Nanofüllstoff.

Für das Nanocomposite gelten die obigen Ausführungen im Zusammenhang mit dem Formteil für den Gasaustausch und die Abtrennung biologischen Materials. Dabei sind das Polymer und der Nanofüllstoff so aufeinander abgestimmt, daß das Formteil eine Permselektivität für bestimmte Gase oder Flüssigkeiten aufweist, so daß diese mit Hilfe der Membran oder des Formteils aus einem Gasgemisch, aus einem Gemisch von Gasen und Flüssigkeiten, aus einem Gemisch von Gasen und Feststoffen, von Flüssigkeiten und Feststoffen oder von Flüssigkeitsgemischen abtrennbar sind.

Generell haben alle Nanocomposites aus einem Thermoplasten und einem Nanofüllstoff alle Vorteile der thermoplastischen Verarbeitung. Diese kann insbesondere durch Spritzguß, Extrusion, Spritzprägen, Mikrostrukturierung usw. erfolgen. Im Stand der Technik waren bislang nur sehr dünnwandige und aufwendig herzustellende Membranen mit entsprechenden Eigenschaften bekannt, die für Form- und Funktionsteile, bei denen hohe Dimensionsstabilität, Steifigkeit usw. gefordert ist, nicht bzw. nur als eingeschweißte Teilkomponenten verwendbar sind.

Zudem sind beispielsweise PP/POSS-Composites hochtransparent, so daß kein optischer Qualitätsverlust eintritt.

Ein Vorteil von Nanocomposites aus einem Thermoplasten und POSS gegenüber Silikon ist in den wesentlich besseren mechanischen Eigenschaften zu sehen, die beispielsweise eine Zentrifugation der Gefäße ermöglichen. Das ist mit vergleichbaren Artikeln aus Silikon nicht erreichbar. Andere vorteilhafte Eigenschaften sind:

Verbesserung der mechanischen und thermischen Eigenschaften gegenüber dem reinen Thermoplasten, Erhaltung oder Erhöhung der Transparenz (bei PP). Weiterhin lassen sich über den Anteil an POSS im Compound gezielt bestimmte Bakterienvermehrungsraten einstellen und somit für nachfolgende Verarbeitungsschritte wie Zentrifugation oder Filtration optimieren. Dies ist mit Silikon bzw. Kernspurenmembranen nicht möglich.

Zudem werden durch den Zusatz von POSS-Derivaten zu Thermoplasten deren Verarbeitungseigenschaften verbessert, da das POSS-Additiv aufgrund der Dispersion auf der molekularen Ebene die Viskosität herabsetzt.

Das Formteil ist ein Behältnis, insbesondere Behältnis für den Laborbedarf mit mindestens einer Aufnahme, die ganz oder teilweise von der Wand begrenzt ist. Gemäß einer weiteren Ausgestaltung ist das Behältnis ein Einzelgefäß (z.B. Reaktionsgefäß oder Reagenzgefäß) oder ein Gefäßstreifen oder eine Mikrotiterplatte oder eine Membranplatte. Die Gefäße können im übrigen bekannten Gefäßen aus dem Laborbereich entsprechen, was die Durchführung des Gasaustauschs und die Abtrennung biologischen Materials und die Stofftrennung erheblich erleichtert. Für den Gasdurchgang besonders vorteilhaft ist, daß die gesamte Oberfläche des Gefäßes für den Gasaustausch genutzt werden kann. Klassische Konzepte von Gefäßen, Gefäßstreifen, Mikrotiterplatten, Deep Well Platten (große Probenzahl, automatisierter Prozeß) und Membranplatten lassen sich mit Nanocomposites durch einfachen Spritzguß verwirklichen. Die Gefäße können insbesondere als Einmalartikel ("Disposables") ausgeführt sein.

Besteht ein Verschluß oder beinhaltet er eine Resealing Mat (eine sich nach dem Durchstechen mit einer Kanüle selbst wiederverschließende Abdeckung), kann das Gefäß ohne Öffnen des Verschlusses mit einer Kanüle befüllt und wieder entleert werden. Der Inhalt des Gefäßes kommt praktisch nicht mehr mit der Außenwelt in Berührung und umgekehrt. Ein Gefäß mit Resealing Mat-Abdeckung ermöglicht auch ein anaerobes Bakterienwachstum, wenn das Gefäß in einer entsprechenden sauerstofffreien Atmosphäre (CO₂ u.a.) gelagert wird.

Darüber hinaus lassen sich Petrischalen mit herkömmlicher Form aus dem Nanocomposite so verwirklichen, daß nach Aufsetzen eines Deckels eine Dichtigkeit gegenüber möglichen Kontaminationen vorhanden ist. Die Abdichtung kann durch einen Schraubverschluß bzw. eine genaue Deckelpassung gefördert werden. Hierdurch werden Handling und Transport von Proben erheblich vereinfacht.

Die Erfindung wird nachfolgend anhand der anliegenden Zeichnung eines Ausführungsbeispieles näher erläutert. In den Zeichnungen zeigen:

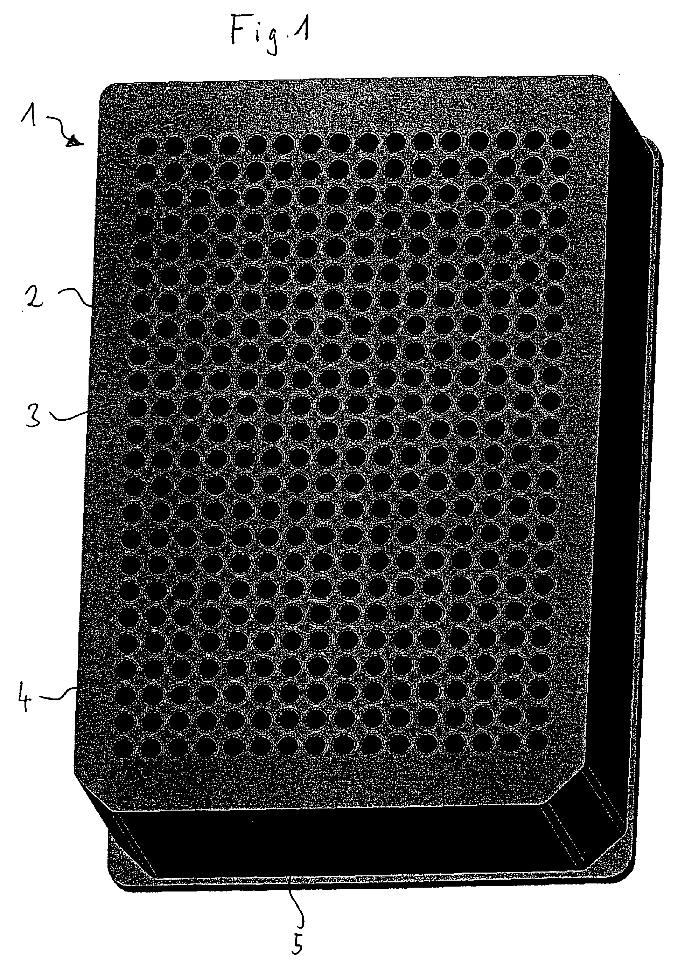

- Fig. 1: eine Mikrotiterplatte in perspektivischer Draufsicht;

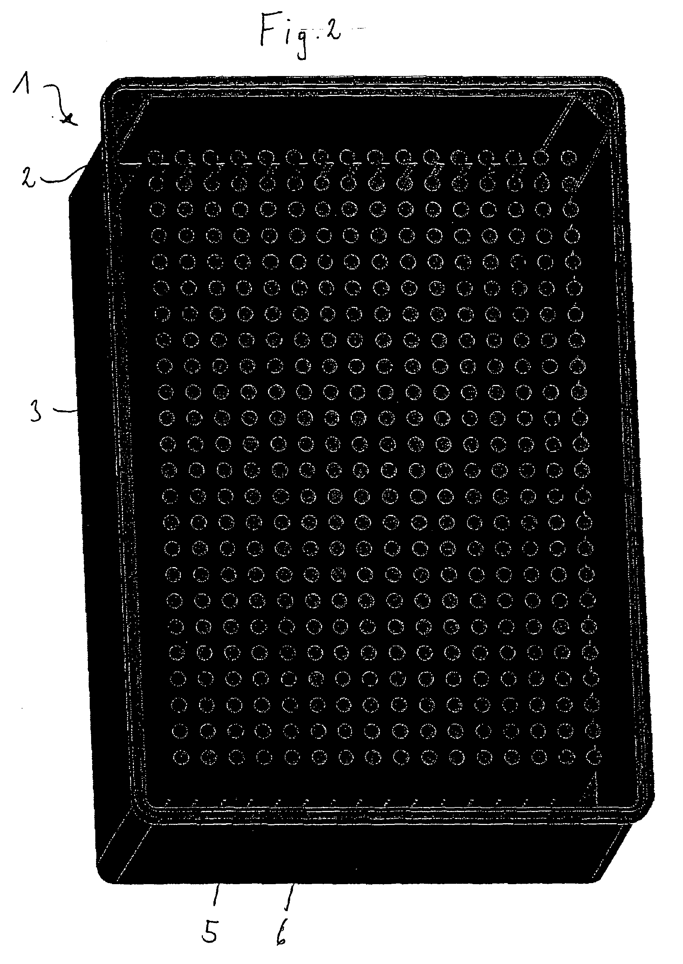

- Fig. 2: dieselbe Mikrotiterplatte in einer Perspektivansicht von unten.

Die Mikrotiterplatte 1 besteht aus einem Rahmen 2 und einer Vielzahl Aufnahmen 3. In 16 Spalten und 22 Reihen sind insgesamt 384 Aufnahmen 3 vorhanden.

Der Rahmen 2 weist eine im wesentlichen rechteckige Platte 4 auf, um deren äußeren Rand eine Einfassung 5 umläuft, die etwa senkrecht von der Unterseite der Platte 4 vorsteht, und zwar etwa bis zum Boden der Aufnahme 3 oder etwas darüber hinaus. Unten hat die Einfassung 5 in bekannter Weise eine Aufweitung 6, die eine Stapelbarkeit auf der Oberseite einer entsprechenden Mikrotiterplatte 1 gewährleistet.

Die gesamte Mikrotiterplatte 1 ist aus einem Thermoplast/POSS-Compound gespritzt. Der Thermoplast ist beispielsweise PP und das POSS-Derivat ist in einem Anteil von 10 % zu dem PP-Basismaterial zugesetzt.

Hierdurch wird eine Steigerung der Gasdurchlässigkeit des Basismaterials bis um den Faktor 10.000 erreicht, wobei zugleich sichergestellt ist, daß Mikroorganismen und Biomoleküle nicht durch die Aufnahmen 3 hindurchtreten können. Die gesamte Wand der Gefäße wird für den Gasdurchgang genutzt.

Durch Aufsetzen einer Resealing-Mat auf die oberen Öffnungen der Aufnahmen 3 kann eine vollständige Abdichtung der Gefäße erreicht werden. Zum Eingeben und Abziehen der Proben wird die Resealing-Mat mit einer Spritze durchstochen.

## Patentansprüche

1. Formteil für den Gasaustausch und die Abtrennung von Mikroorganismen und/oder Biomolekülen mit mindestens einer Wand bestehend zumindest teilweise aus einem gasdurchlässigen und für Mikroorganismen und/oder Biomoleküle undurchlässigen Nanocomposite enthaltend mindestens ein Polymer und mindestens einen Käfigmoleküle umfassenden Nanofüllstoff, das ein Behältnis mit mindestens einer eine Öffnung aufweisenden Aufnahme ist, die ganz oder teilweise von der Wand begrenzt ist, so daß es einen Gasaustausch ermöglicht, der nicht durch die Öffnung hindurchgeht.

2. Formteil nach Anspruch 1, das ein Einzelgefäß oder ein Gefäßstreifen oder eine Mikrotiterplatte oder eine Membranplatte oder eine Petrischale oder ein künstliches Atmungsorgan ist.

3. Formteil nach Anspruch 1 oder 2, bei dem die Öffnung von einem Verschluß verschlossen ist.

4. Formteil nach Anspruch 3, bei dem der Verschluß eine mittels einer Nadel durchstechbare Wand aufweist, die sich nach dem Durchstechen wieder schließt.

5. Formteil nach Anspruch 4, bei dem der Verschluß aus Silikon ist.

6. Formteil nach einem der Ansprüche 1 bis 5, bei dem das Polymer ein Thermoplast oder Elastomer oder Duromer und/oder der Nanofüllstoff ein polyhedraler Oligosilsesquioxan mit organischer Seitengruppe ist.

## Claims

1. A moulded component for a gas exchange and separation of microorganisms und/or biomolecules, which includes at least one wall and at least partially consists of a nanocomposite permeable to gas and impermeable to microorganisms and/or biomolecules, containing at least one polymer and at least one nanofiller comprising cage molecules, which is a case including at least one receptacle with an aperture which is bordered wholly or partially by the wall so as to enable a gas exchange which does not pass through the aperture.

2. The moulded component according to claim 1 which is an individual well or a strip of wells or a microtitration plate or a diaphragm plate or a Petri culture dish or an artificial respiratory organ.

3. The moulded component according to claim 1 or 2 wherein the aperture is closed by a closure.

4. The moulded component according to claim 3 wherein the closure has a wall which can be pierced by a needle and will close again after piercing.

5. The moulded component according to claim 4 wherein the closure is made of silicone.

6. The moulded component according to any one of claims 1 to 5 wherein the polymer is a thermoplast or elastomer or duromer und/or the nanofiller is a polyhedral oligosil sesquioxane having an organic side group.

## Revendications

1. Pièce moulée pour l'échange gazeux et la séparation de micro-organismes et/ou de biomolécules, munie d'au moins une paroi se composant au moins en partie d'un nanocomposite perméable aux gaz et imperméable aux micro-organismes et/ou aux biomolécules, ledit nanocomposite comprenant au moins un polymère et au moins une matière de remplissage de taille nanométrique comprenant une molécule cage, ladite pièce étant un récipient doté d'au moins un élément récepteur comportant une ouverture, ledit élément récepteur étant délimité entièrement ou partiellement par la paroi, de sorte que ladite pièce permet un échange gazeux qui ne passe pas par l'ouverture.

2. Pièce moulée selon la revendication 1, ladite pièce étant un récipient individuel ou une bande de récipients ou une plaque microtitre ou une plaque à membrane ou une boîte de Petri ou un organe respiratoire artificiel.

3. Pièce moulée selon la revendication 1 ou 2, dans laquelle l'ouverture est fermée par un obturateur.

4. Pièce moulée selon la revendication 3, dans laquelle l'obturateur comporte une paroi pouvant être perforée à l'aide d'une aiguille, ladite paroi se refermant après la perforation.

5. Pièce moulée selon la revendication 4, dans laquelle l'obturateur est en silicone.

6. Pièce moulée selon l'une des revendications 1 à 5, dans laquelle le polymère est une matière thermoplastique ou un élastomère ou un duromère et/ou la matière de remplissage de taille nanométrique est un silsesquioxane oligomérique polyédrique avec un groupe latéral organique.
